# EUROPEAN PATENT APPLICATION

(11) **EP 1 661 590 A1**
(43) Date of publication of application: **31.05.2006**
(21) Application number: 04703330.3
(22) Date of filing: 20.01.2004
(51) Int. Cl.: A61M 1/06

(54) **MULTIFUNCTIONAL MAMMARY-GLAND-MERIDIAN FREQUENCY SPECTROMETER**

(30) Priority: 08.08.2003 CN 03209609
(71) Applicant: Toppor Healthtec, Inc., Zhejiang 315040 (CN)
(72) Inventor: FEI, Daowei, Ningbo, Zhejiang 315208 (CN)
(74) Representative: Bloch, Gérard
(86) International application number: PCT/CN2004/000073
(87) International publication number: WO 2005/014081

(57) **Abstract**

A kind of multifunctional mammary-gland-meridian frequency spectrometer, including: a whole milk bra, comprising inner and outer bras; a electric heater inserted between said inner and outer bras; permanent magnets, mounted between said inner bra and electric heater; magneto-electric poles, formed with conductive rubbers, which are embedded on the inner bra equably. An electronic impulse spectrum generator is connected to the electric heater, the permanent magnets reeled with windings and the magneto-electric poles of conductive rubbers. An electric vibrator or a suck tube connected with a electric vibrator, mounted in the center of the front of said bra. A nipple fixture is provided at the front-end of the suck tube, which are a circuit of inward projections. The invention is used to apply the constant frequency spectrum electromagnetic pulse to the meridian of the breasts, to perform the heat treatment for the breast with constant temperature, to provide vibratory massage and vacuum sucking, which is effective for the precautions against mastitis, crater nipple and lobular hyperplasia, etc.

## Description

### Filed of the Invention

The present invention relating to a multifunctional mammary-gland-meridian frequency spectrometer belongs to medical treatment and health care products.

### Background of the Invention

Lying-in women during the lactation always have troubles in milk filled up or milk less. It is always happened for this group of women that breasts are floppy and atrophy, sizes of two breasts are different and breast has lobular hyperplasia etc. Therefore, they need a perfect medical treatment and health care products. This kind of products are many in market now, their functions are almost the same, to suck the extra milk out by a negative pressure to achieve the purpose of dredging mammary gland and prevention and cure of mastitis. However, this kind of products is not effective for patients who have had the disorder of milk filled up or mastitis or lobular hyperplasia already. In addition, this kind of devices is useless in improving the symptoms of breast floppy and size different. The Chinese patent No. 01255040.X titled "Multifunctional device for dredging mammary-gland and breast health" disclosed an invention, which can foment and massage breast and possesses functions of dredging meridian and attempering qi and blood, is benefit for the breast health card and augment if use it regularly. However, this kind of device needs manual operating on a sucking ball for creating negative pressure, it is not convenient for use. Its massages can not on suitable points of the breast, thereby the cure result is not good. To date, there is not a physical therapy device that can be imitative of acupuncture by electromagnetic pulse produced from the frequency spectrometer to act on points of mammary-gland-meridian for cure breast diseases. Consequently, people are looking for a multifunctional mammary-gland-meridian frequency spectrometer for treatment of different breast diseases.

### Summary of the Invention

The purpose of the present invention is to provide a multifunctional mammary-gland-meridian frequency spectrometer that can be imitative of acupuncture by electromagnetic pulse produced from the frequency spectrometer to act on points of mammary-gland-meridian for curing breast diseases and caring breast health.

The purpose of the present invention is realized by that a multifunctional mammary-gland-meridian frequency spectrometer comprises a whole milk bra including inner and outer bras, the outer bra covers and connects the inner bra; a electric heater is inserted between the inner and outer bras; permanent magnets are mounted between the inner bra and the electric heater; magneto-electric poles formed with conductive rubbers are embedded on the inner bra equably. An electronic impulse spectrum generator is connected to the electric heater, the permanent magnets reeled with windings and the magneto-electric poles of conductive rubbers. An electric vibrator is mounted between the inner and outer bras in the center of the front of the bra, vent pipe and outlet hole are located under the electric vibrator. A hole is opened at the center of the front of the bra, a suck tube is located before and connected with the hole. The electric vibrator is mounted before the suck tube, a milk packet located under the suck tube is connected with the suck tube. A nipple fixture which is a circuit of inward projection is provided at the front-end of the suck tube. The two sides of the milk bra body appears arced bulge.

Comparing the present invention with the prior art, the multifunctional mammary-gland-meridian frequency spectrometer can be imitative of acupuncture by electromagnetic pulse produced from the frequency spectrometer to act on points of mammary-gland-meridian for curing breast diseases and breast health care; it also can perform heat treatment, vibration massage and vacuum sucking to breast. The present invention can provide precaution against mastitis, crater nipple and lobular hyperplasia, etc.

### Brief Description of the Drawings

Fig. 1 is a schematic figure for the structure of the present invention.
Fig. 2 is a schematic figure for the structure of the another embodiment of present invention.

### Detail Description of the Invention

### Embodiment 1

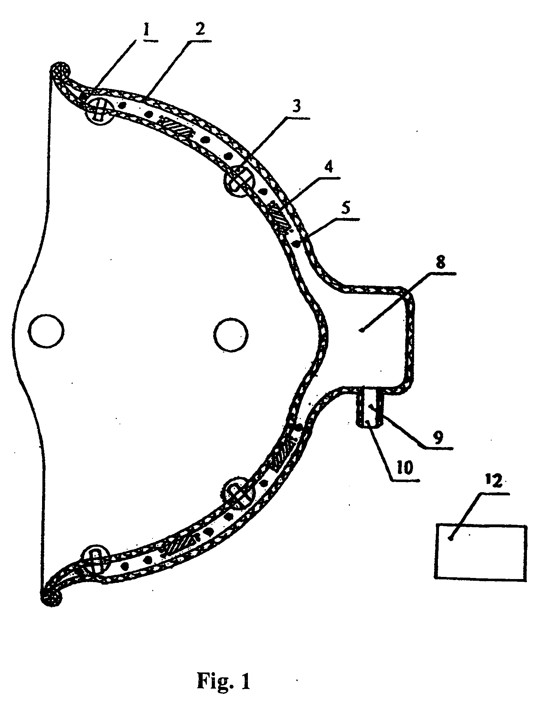

As showing in the Fig. 1, the multifunctional mammary-gland-meridian frequency spectrometer has a whole milk bra with evase shape including inner bra 1 and outer bra 2, the outer bra 2 covers on and connects with the inner bra 1, The two sides of the milk bra body appears arced bulge. An electric heater 5 controlled by an invariable electronic circle is inserted between the inner bra 1 and outer bra 2, the electric heater 5 is made from electric heating wire or electric heating layer or electric PCT ceramics heating materials. Permanent magnets 4 that are wined with coils for creating alternative magnet filed are mounted between the inner bra 1 and outer bar 2 and between electric heaters 5. magneto-electric poles formed with conductive rubbers are embedded on the inner bra 1 equably, the part body of the magnet-electric poles 3 is embedded between inner bra 1 and outer bra 2, the other part body comes out of the out side of the inner bra 1 as a protrusion. An electric vibrator 8 is mounted between the inner bra 1 and outer bra 2 in the center of the front of the bra. A vent pipe 9 located under the electric vibrator 8 is connected with an electric vacuum pump which is located in controlling box 12 through a inspiration pipe. Wires of magnet-electric poles 3, permanent magnets 4, electric heater 5, and electric vibrator 8 are through an outlet hole 10 to connect with an electronic impulse spectrum generator which is located in the controlling box 12. When operation first connect the device with power source, set its output voltage as 6 V, 12 V, 24 V and 32 V, then it can process imitative acupuncture by electromagnetic pulse produced from the frequency spectrometer to act on points of mammary-gland-meridian; also it can process alternative magnetic treatment, heating treatment, vibration massage, vacuum sucking. The present invention is effective for the precautions against mastitis, crater nipple and lobular hyperplasia, etc. It also can improve symptoms of breasts floppy and atrophy if using it regularly.

### Embodiment 2

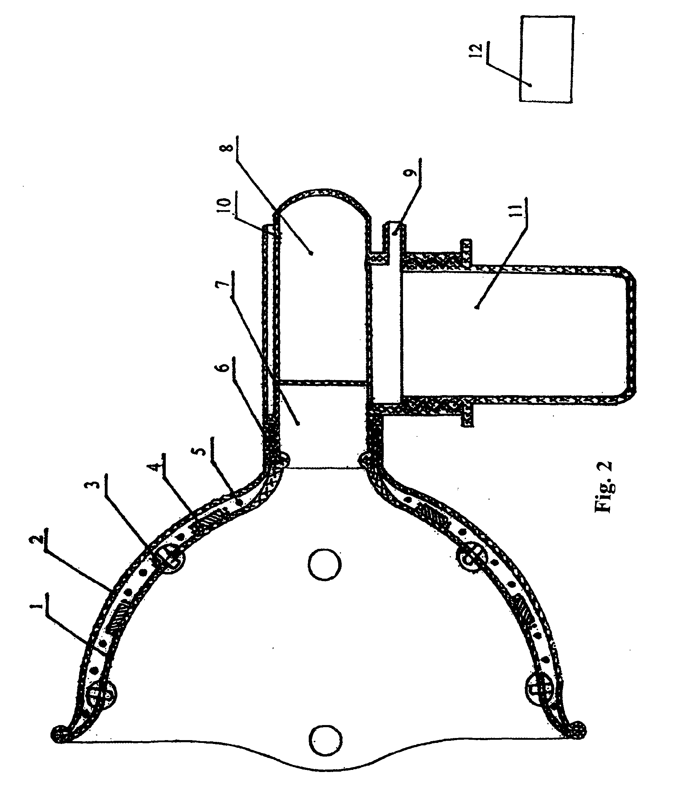

As showing in Fig. 2, a hole is opened at the center of the front of the bra, a suck tube 7 is located before and connected with the hole by screw thread or bond. A nipple fixture 6 which is a circuit of inward projection is provided at the front-end of the suck tube 7. The bottom end of the suck tube 7 is connected with a milk packet 11 by screw thread connection, a milk-out-hole of the suck tube 6 is through the milk packet 11. An electric vibrator 8 is fixed on the front of the suck tube 6.
A vent pipe 9 located the side end of the bottom of the suck tube 7 is connected with an electric vacuum pump which is located in controlling box 12 through a inspiration pipe. Because of the negative pressure produced by the electric vacuum pump and the massage produced by the electric vibrator 8 the crater nipple can be pulled out, the filled up milk can be sucked out to the milk packet 11 through the milk-out-hole of the suck tube 6, as a result the mammary gland is unimpeded.

When operation first connect the device with power source, then the device processes imitative acupuncture by electromagnetic pulse produced from the frequency spectrometer to act on points of mammary-gland-meridian, alternative magnetic treatment, heating treatment, vibration massage, vacuum sucking. The rest part of the structure is the same as the embodiment 1. The present invention is effective for the precautions against mastitis, crater nipple and lobular hyperplasia, etc. It also can improve symptom of breasts floppy and atrophy if using it regularly. It is an ideal physical therapy device for breast medical treatment and health care.

## Claims

1. A multifunctional mammary-gland-meridian frequency spectrometer comprising:
whole bra including inner bra and outer bra, said outer bra covering on and connecting with said inner bra;
an electric heater be located between said inner bra and outer bra;
permanent magnets wined with coils be mounted between said inner bra and outer bra and between said electric heaters;
magneto-electric poles formed with conductive rubbers be embedded on said inner bra equably;
an electronic impulse spectrum generator be connected to said electric heater, said permanent magnets reeled with windings and said magneto-electric poles of conductive rubbers.

2. The spectrometer of claim 1, wherein an electric vibrator is mounted between the inner and outer bras in the center of the front of the bra.

3. The spectrometer of claim 2, wherein a vent pipe and an outlet hole are located under said electric vibrator.

4. The spectrometer of claim 1, wherein a hole is opened at the center of the front of said bra, a suck tube is located before and connected with said hole, a vibrator is fixed on the front end of said suck tube, a milk packet is located under and connected with said suck tube.

5. The spectrometer of claim 4, wherein a nipple fixture which is a circuit of inward projection is provided at the front-end of the suck tube.

6. European Region Phase European Region Phase European Region Phase The spectrometer of claim 1 or 2 or 3 or 4, wherein the two sides of said bra body appear arced bulge.
